# NEUE EUROPÄISCHE PATENTSCHRIFT

(11) **EP 0 152 544 B2**
(45) Veröffentlichungstag und Bekanntmachung des Hinweises auf die Entscheidung über den Einspruch: **08.03.1995**
(45) Hinweis auf die Patenterteilung: 16.10.1991
(21) Anmeldenummer: 84113540.3
(22) Anmeldetag: 09.11.1984
(51) Int. Cl.: A61L 2/26

(54) **Sterilisierbehälter**
Sterilization container
Récipient de stérilisation

(30) Priorität: 11.11.1983 DE 3340963; 19.10.1984 DE 3438463
(43) Veröffentlichungstag der Anmeldung: 28.08.1985
(73) Patentinhaber: Georg Wagner KG, 80634 München (DE)
(72) Erfinder: Lorenz, Jürgen, D-8000 München 70 (DE); Wagner, Hans, D-8000 München 22 (DE)
(74) Vertreter: Koch, Günther, Dipl.-Ing.

(56) Entgegenhaltungen:
- EP-A- 0 080 203
- DE-U- 1 757 714
- FR-A- 2 375 869

## Beschreibung

Die Erfindung bezieht sich auf einen Sterilisierbehälter der im Oberbegriff des Patentanspruchs 1 angegebenen Gattung. Ein solcher Sterilisierbehälter ist aus der DE-PS 31 46 349 und der entsprechenden EP-A-0 080 203 bekannt. Dieser Sterilisierbehälter ist bei allseitig geschlossenem Aufbau und geschützten Ventilen bzw. Filtern mit seinem Behälternutzraum gegen die Außenluft hygienisch abgedichtet und gewährleistet eine sterile Lagerung des Behälterinhaltes mit einer günstigen Dampfverteilung, wobei die Dampfdurchtrittsöffnungen an Stellen zu liegen kommen, die über einen labyrinthförmigen Strömungspfad nach dem Zwischendeckel führen, so daß mechanische Verletzungen des Filters bzw. der Ventile ausgeschlossen sind.

Bei diesem bekannten Sterilisierbehälter sind Behälterdeckel und Zwischendeckel als Baueinheit ausgeführt, und sie werden stets gemeinsam auf das Unterteil aufgesetzt und mit diesem durch Verschlüsse verspannt und ebenso gemeinsam nach Lösen der Verschlüsse abgenommen. Im abgenommenen Zustand kann zwar der Zwischendeckel vom Behälterdeckel gelöst werden, jedoch ist diese Trennung nicht möglich solange der Sterilisierbehälter geschlossen ist. Dies bedingt, daß der Sterilisierbehälter mitsamt der aus Zwischendeckel und Behälterdeckel bestehenden Einheit in den OP-Raum überführt werden müssen. Dies kann dazu führen, daß auf der Oberseite des Behälterdeckels anhaftender Staub möglicherweise mit Keimen in den OP-Raum verschleppt wird und unter ungünstigen Umständen mit sterilen Gegenständen in Berührung gebracht. Diese Deckeloberfläche ist praktisch die einzige Fläche wo in Lagerräumen abgestellte Sterilisierbehälter mit Staub oder Keimen behaftet werden können.

Der Erfindung liegt daher die Aufgabe zugrunde, einen gattungsgemäßen Sterilisierbehälter derart auszubilden, daß diese Gefahr der Kontamination ausgeschlossen wird.

Gelöst wird die gestellte Aufgabe durch die im Kennzeichnungsteil des Patentanspruchs 1 angegebenen Merkmale.

Durch die Erfindung wird erreicht, daß der möglicherweise kontaminierte Behälterdeckel überhaupt nicht in den Operationssaal überführt zu werden braucht, sondern vor Erreichen des Operationssaales abgelegt werden kann, ohne daß dadurch die Gefahr einer Rekontamination des Behälterinhalts erfolgen kann, weil dieser Inhalt durch den Zwischendeckel der Behälterunterteile keimfrei verschlossen bleibt und vor seiner Öffnung weiter behandelt und transportiert werden kann, ohne daß eine Kontamination zu befürchten wäre.

Ein besonders sicherer Schutz gegen eine Rekontamination ergibt sich wenn gemäß den Merkmalen des Anspruchs 2 die Anordnung so getroffen ist, daß die Verschlüsse des Zwischendeckels erst dann zugänglich werden, wenn der Behälterdeckel abgenommen ist. Hierdurch wird mit Sicherheit verhindert, daß außerhalb des OP-Raums versehentlich der Behälterdeckel zusammen mit dem Zwischendeckel abgenommen und der Behälterinhalt auf diese Weise freigelegt wird.

Dadurch, daß der Behälterdeckel nicht mehr mit dem Zwischendeckel verbunden ist, ergibt sich ein weiterer Vorteil, weil bei Verwendung eines Einwegfilters nach Abnahme des Zwischendeckels dieser freiliegt und sofort vernichtet werden kann, so daß nicht die Gefahr einer nochmaligen Verwendung besteht.

Bei gattungsgemäßen Sterilisierbehältern und allen anderen bekannten Sterilisierbehältern, die mit Filterblättern in Gestalt von Dauerfiltern oder Einwegfiltern arbeiten, werden diese Filter während des Dampf-Sterilisationsvorganges von den Medien durchströmt. Dies ist deshalb notwendig, weil die an sich nur zur Verhinderung der Reinfektion nach der Sterilisation bis zur Bereitstellung am Operationstisch erforderlichen Filter nicht nachträglich nach vollendeter Sterilisation zur Abdeckung der zuvor offenen Deckelöffnungen angebracht werden können, weil eine solche nachträgliche Filterbefestigung unter Aufrechterhaltung der geforderten Keimfreiheit nicht möglich ist.

Die üblichen früher benutzten Filter ermöglichen einen langsamen oder mäßig schnellen Durchtritt von Dampf und Luft in beiden Richtungen. Neuerdings werden jedoch in der Dampf-Sterilisation Druckdifferenzverfahren angewandt, bei denen der Druck beschleunigt auf hohe Werte ansteigt, so daß sich eine hohe Stoßbelastung der Filter ergibt. Gleichzeitig werden verschärfte Anforderungen an die Dichtigkeit der Filtermaterialien bei Sterilisierbehältern gestellt, wobei eine Porendichte von etwa 0,5 um notwendig ist.

Diesen beiden Forderungen, die moderne Sterilisationsverfahren mit sich bringen, sind die bisher bekannten Sterilisationsbehälter mit Filtern häufig nicht gewachsen. Der Strömungswiderstand der Filter verzögert den Druckausgleich zwischen der Sterilisierkammer und dem Behälterinnenraum, und dies führt zum Aufbau eines Überdrucks an den Behälteraußenflächen. Dies hat bei bekannten Behältern zur Folge, daß ein Druckausgleich auf dem Wege der Deformation des Sterilisationsbehälters erfolgt, d.h. es erfolgt eine Implosion, die in der Regel irreparabel ist. Bei Auftreten derartiger Druckstöße kann auch das zur Abstützung des Filters benutzte Filterhalteblech über die Elastizitätsgrenze hinaus deformiert werden und dadurch die Fähigkeit verlieren, den Filter dichtend an den Deckel anzupressen. Dies birgt insofern eine große Gefahr in sich, als diese Deformation nicht ohne weiteres erkennbar wird und dann bis zur Bereitstellung des Sterilisierbehälters im Operationssaal ein nicht bemerkter Nebenschlußpfad am Filter vorbei vorhanden ist, der diesen Filter praktisch wertlos macht.

Die Erfindung sieht daher weiter vor, einen Sterilisierbehälter derart zu verbessern, daß auch bei Benutzung sehr feinporiger Filter und beschleunigten Druckanstiegen eine Beschädigung von Sterilisierbehältern, Filter oder Filterhalteblech vermieden und die Dichtigkeit des Sterilisierbehälters nach erfolgter Sterilisation gewährleistet wird. Dies wird dadurch erreicht, daß das Filterblatt beim Überschreiten eines vorbestimmten Druckdifferentials von den Deckelöffnungen unter Freigabe eines Nebenstrompfades ventilartig abhebbar ist und nach Unterschreiten eines vorbestimmten Druckdifferentials die Deckelöffnungen erneut schließt.

Dadurch, daß ein Nebenstrompfad geschaffen wird, der während der Sterilisation den Medienaustausch ermöglicht, wird eine unzulässig hohe Druckbeaufschlagung von Filter und Halteelementen mit Sicherheit verhindert. Gleichzeitig gewährleisten die zur zeitweisen Schaffung des Strömungs-Nebenschlußpfades benutzten Federelemente einen sicheren Verschluß dieser Nebenstrompfade nach Abschluß der Sterilisation, so daß die Wirksamkeit der Filter voll erhalten und gewährleistet bleibt.

Dieser Sicherheitsventilaufbau kann für alle mit Filter ausgerüstete Sterilisierbehälter unabhängig von deren sonstigem Aufbau Anwendung finden.

Weitere zweckmäßige Ausgestaltungen der Erfindung ergeben sich aus den Unteransprüchen.

Nachstehend werden Ausführungsbeispiele der Erfindung anhand der Zeichnung beschrieben. In der Zeichnung zeigen:
Fig. 1 einen Vertikal schnitt eines erfindungsgemäß ausgebildeten, mit Dauerfilter versehenen Sterilisierbehälters;
Fig. 2 eine der Figur 1 entsprechende Schnittansicht eines erfindungsgemäßen Sterilisierbehälters mit Einwegfilter;
Fig. 3 eine den Figuren 1 und 2 entsprechende Schnittansicht eines erfindungsgemäß ausgebildeten Sterilisierbehälters mit einem Doppelventil zum Medienaustausch;
Fig. 4 einen Vertikalschnitt eines Sterilisierbehälters mit am perforierten Behälterdeckel angeordnetem Filterblatt;
Fig. 5 einen Vertikalschnitt eines Sterilisierbehälters mit am vom Behälterdeckel umschlossenen Zwischendeckel angeordnetem Filterblatt.

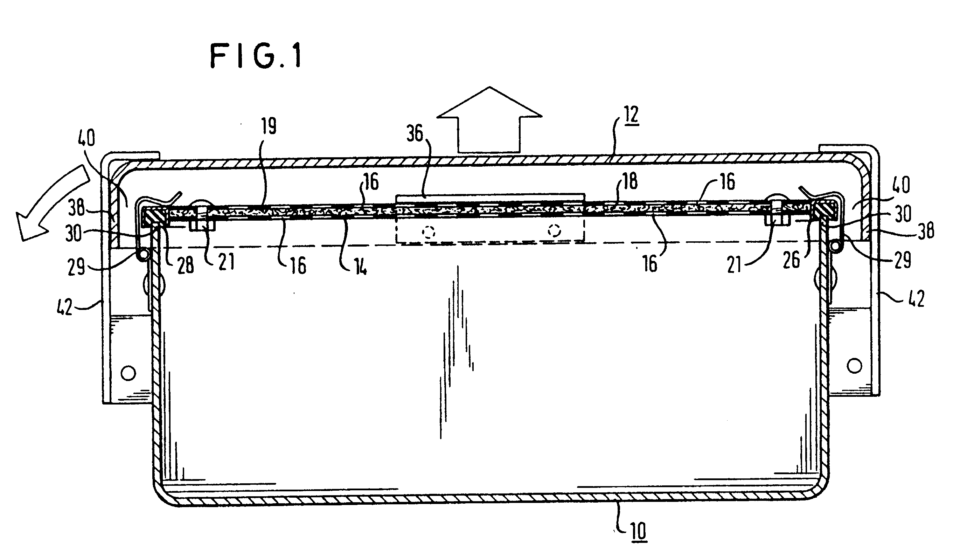

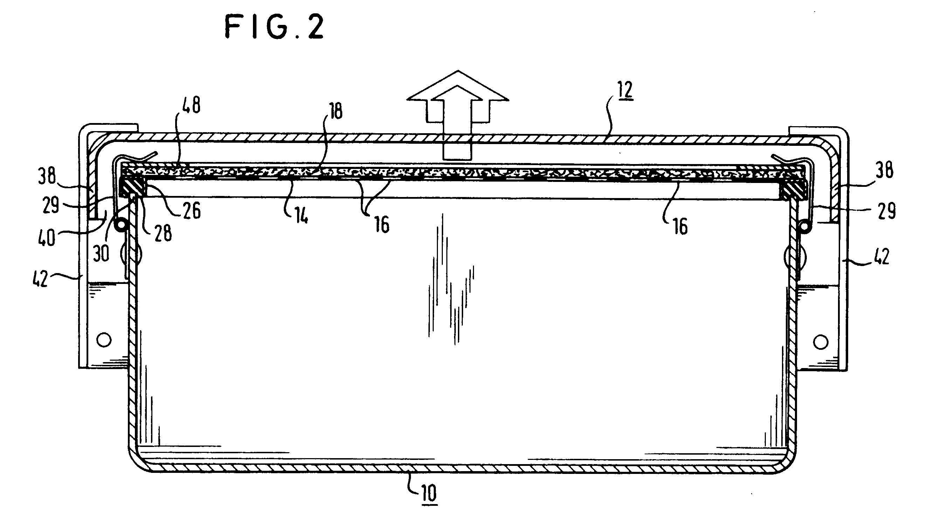

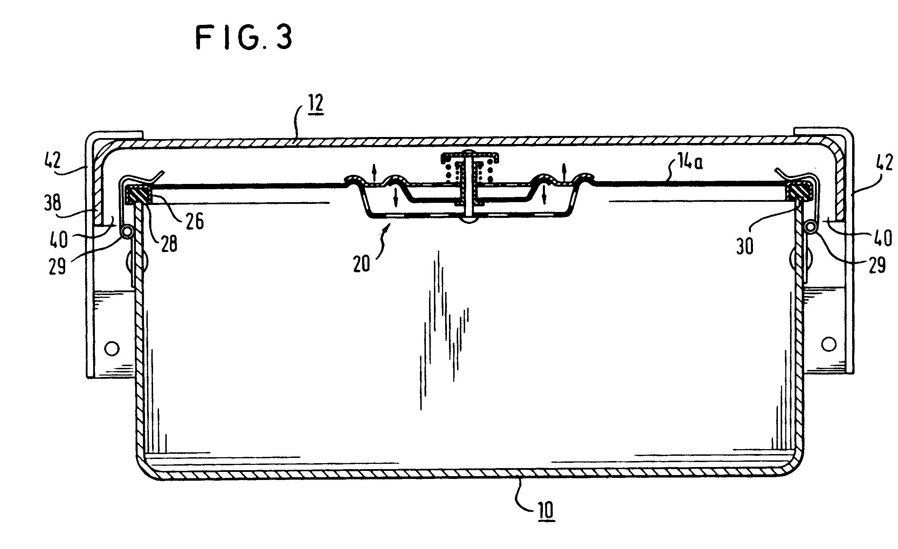

Die in den Fig. 1 bis 3 dargestellten Sterilisierbehälter sind entsprechend den Lehren der DE-PS 31 46 349 bzw. der entsprechenden EP-A-80203 ausgebildet und sie bestehen aus einem Behälterunterteil 10, einem Behälterdeckel 12 und einem Zwischendeckel 14. Gemäß dem Ausführungsbeispiel nach Fig. 1 und 2 ist der Zwischendeckel 14 mit Perforationslöchern 16 versehen, und diese sind durch ein Filter 18 abgedeckt, das bei dem Ausführungsbeispiel nach Fig. 1 als Dauerfilter ausgebildet ist, während es bei dem Ausführungsbeispiel nach Fig. 2 als Einwegfilter ausgebildet ist. Bei dem Ausführungsbeispiel nach Fig. 3 ist in den nicht perforierten Zwischendeckel 14a ein Doppelventil 20 eingebaut, welches zweckmäßigerweise von der Bauart gemäß DE-AS 12 17 550 ist. Stattdessen können auch zwei Ventile entsprechend der DE-AS 12 17 551 in den Zwischendeckel 14a eingebaut sein.

Bei dem Ausführungsbeispiel nach Fig. 1 ist das Filter 18 durch ein perforiertes Filterhalteblech 19 abgedeckt, und dieses ist mit dem Zwischendeckel 14 durch Schraubverbindungen 21 verbunden, so daß die Teile 14, 18, 19 eine Baueinheit darstellen. Anstelle der Schraubverbindungen 21 können auch andere lösbare Verbindungen vorgesehen werden.

Bei dem Ausführungsbeispiel nach Fig. 2 wird das Filtertuch 18 durch einen Filterhalterahmen 48 auf den Rand 10 des Zwischendeckels 14 gedrückt.

Bei allen Ausführungsbeispielen weist der Zwischendeckel 14 bzw. 14a einen umgekehrt U-förmigen Endflansch 26 mit einem Dichtring 28 auf. Dieser Dichtring ruht in zusammengebautem Zustand auf dem Oberrand 30 des Behälterunterteils 10. Der dampfbeständige Dichtring 28 dichtet den Behälterinnenraum hermetisch gegen die Außenluft ab. Dieser Dichtring 28 könnte auch auf den Rand 30 des Behälterunterteils 10 aufgesetzt sein und gegen eine entsprechende Dichtfläche des Zwischendeckels 14 wirken.

Der Zwischendeckel 14 bzw. 14a ist über Spannverschlüsse 29 auf dem Behälterunterteil 10 festgelegt, und diese Spannverschlüsse 29 gewährleisten einen Dichtungseingriff zwischen dem Dichtring 28 und dem Behälteroberrand 30. Diese Spannverschlüsse 29, die als Kniehebelverschlüsse, als Schraubverschlüsse oder in anderer Weise ausgebildet sein können, liegen derart unter dem seitlich übergreifenden Behälterdeckel 12, daß sie erst nach Abnahme dieses Deckels geöffnet bzw. geschlossen werden können.

Der Behälterdeckel 12 umschließt mit seinem seitlich heruntergezogenen Deckelflansch 38 seitlich den Oberrand 30 des Behälterunterteils unter Belassung eines Ringspaltes 40, durch den hindurch der Medienaustausch erfolgen kann.

Der Behälterdeckel 12 ist mittels eines Spannverschlusses 42 auf des Behälterunterteil 10 festlegbar. Dabei drücken an der Innenseite des Behälterdeckels 12 fixierte Haltebleche 36 von oben her auf den Rand des Zwischendeckels 14 bzw. 14a, bzw. auf das Filterhalteblech 19 oder den Filterhalterahmen 48, wodurch der Dichtungseingriff, der durch die Spannverschlüsse 29 bewirkt wird, verstärkt wird. Diese Haltebleche, die eine Abstützung des Deckels 12 gegenüber dem Behalterunterteil 10 über den Zwischendeckel bewirken, sind nur in Fig. 1 dargestellt, sie sind jedoch auch bei dem Ausführungsbeispiel nach Fig. 2 und 3 vorhanden.

Durch diese Ausbildung wird gewährleistet, daß der möglicherweise an seiner äußeren Oberfläche mit Staub behaftete Behälterdeckel 12 nach Lösen der Spannverschlüsse 42 unabhängig von dem Zwischendeckel 14 abgenommen und außerhalb des OP-Raumes belassen werden kann. Nach Abnahme dieses als Schutz dienenden Deckels 12 bleibt der Behälter durch den Zwischendeckel immer noch steril abgedichtet, da die Verschlüsse 29 den Zwischendeckel dichtend auf dem Behälterunterteil 10 halten und somit gewährleisten, daß der Inhalt weiter keimfrei verbleibt.

Gemäß den dargestellten Ausführungsbeispielen ist der Behälterdeckel 12 über die Verschlüsse 42 mit dem Behälterunterteil 10 verspannt. Es wäre jedoch auch möglich, den Behälterdeckel 12 über geeignete Verschlüsse mit den Zwischendeckel 14 oder seiner Halterung zu verbinden. Wichtig ist lediglich, daß der Behälterdeckel 12 abgenommen werden kann, ohne daß die Dichtung zwischen dem Zwischendeckel 14 und dem Behälterunterteil 10 ihre Wirksamkeit verliert.

Der Deckel 12 ist sowohl während der Sterilisation als auch während der späteren Lagerung auf dem Unterteil fixiert und wird erst abgenommen, kurz bevor der Behälter in den OP-Raum gebracht wird.

Bei der Sterilisation erfolgt der Medienaustausch: Luft-Dampf über den zwischen Deckelflansch 38 und Oberrand 30 des Behälterunterteils 10 verbleibenden Ringspalt 40, dessen Querschnitt entsprechend dimensioniert ist. Die über den Dekkel-Ringspalt 40 im Überdruck- bzw. Unterdruckzustand zugeführten bzw. abgeführten Medien durchströmen zwangsläufig das Filter 18 bei dem Ausführungsbeispiel gemäß Fig. 1 und 2, bzw. das Ventil 20 bei dem Ausführungsbeispiel nach Fig. 3.

Wichtig ist, daß der Verschluß 29 zwischen Behälterunterteil 10 und Zwischendeckel 14 erst dann geöffnet werden kann, wenn der Behälterdekkel 12 nach Lösen seiner Spannverschlüsse 42 abgehoben ist.

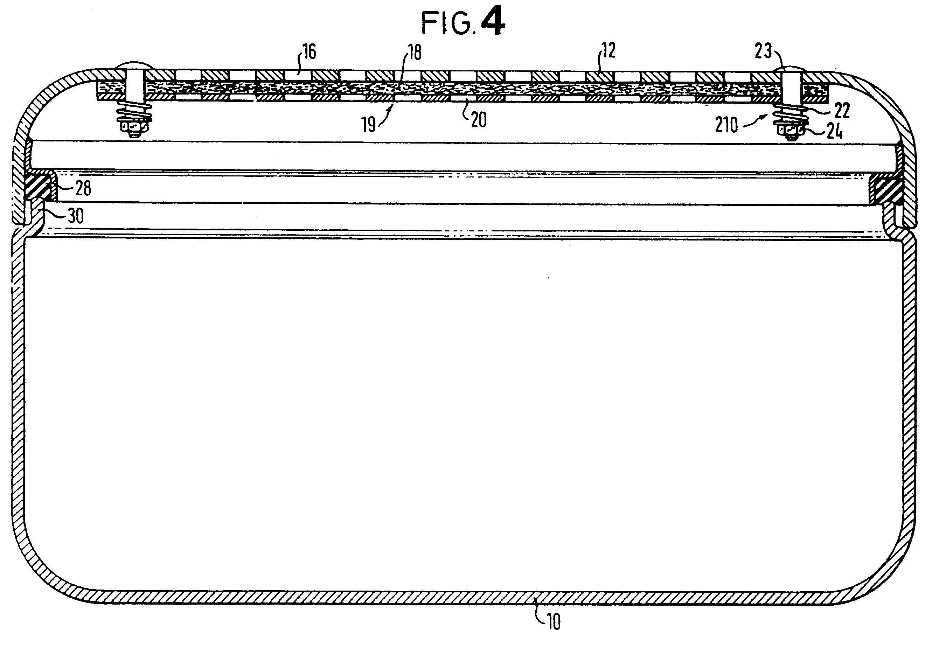

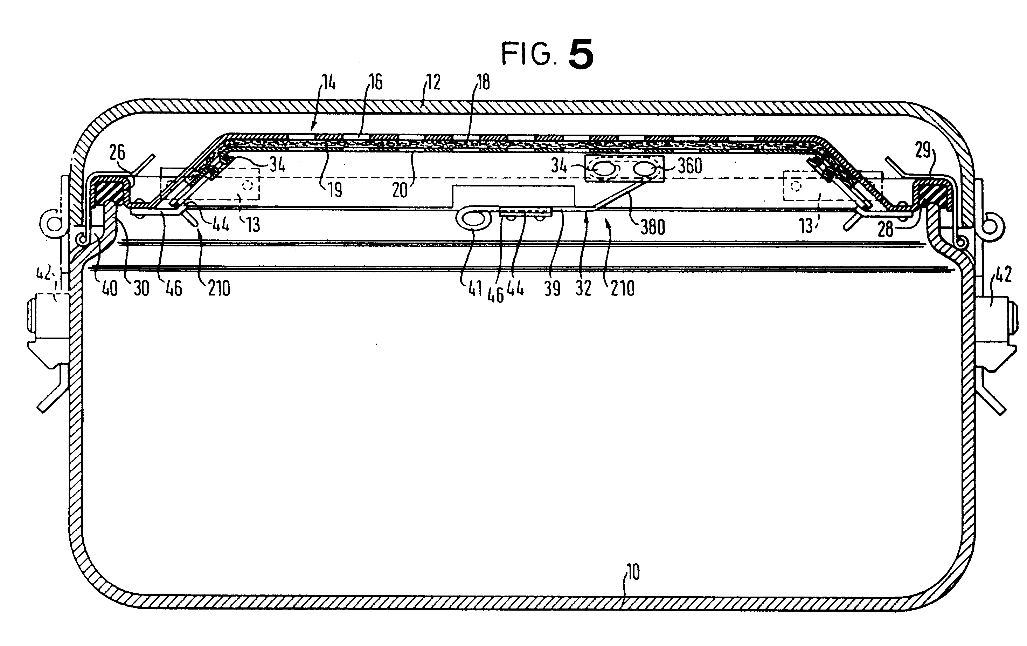

Im folgenden wird auf die Figur 4 und 5 Bezug genommen:
Der Sterilisierbehälter gemäß Figur 4 besteht aus einem Behälterunterteil 10 und einem Behälterdekkel 12, der mit einem umlaufenden Dichtring 28 versehen ist, welcher in Dichtungseingriff mit dem nach innen eingezogenen Oberrand 30 des Behälterunterteils steht, wenn in der Zeichnung nicht dargestellte Spannungsverschlüsse den Behälterdeckel 12 gegen das Unterteil 10 vorspannen. Der Deckel 12 ist mit Öffnungen in Gestalt von Perforationslöchern 16 versehen, die in ebenfalls an sich bekannter Weise von oben her durch eine in der Zeichnung nicht dargestellte Schutzabdeckung geschützt sein können.

Der Innenseite des Deckels 12 liegt ein Filterblatt 18 an, welches als Dauerfilter oder als Einmalfilter aus Papier oder dergleichen ausgebildet sein kann.

Dieses Filterblatt 18 ist von innen her durch ein mit Perforationslöchern 20 versehenes Filterhalteblech 19 derart abgestützt, daß er über die Perforationslöcher 16 und 19 führende Strömungspfad nach Abschluß des Sterilisiervorganges durch das Filterblatt hindurch verlaufen muß. Das Filterhalteblech 19 ist am Deckel 12 mit mehreren durch Löcher von Deckel, Filterblatt und Filterblech geführte Bolzen 23 und darauf aufgeschraubte Muttern 24 gehalten. Durch Federelemente 210wird das Filterhalteblech mit einer vorbestimmten Vorspannung gegen das Filterblatt 18 und die Innenseite des perforierten Deckelteils gedrückt. Die Filterelemente sind nach dem Ausführungsbeispiel als Druckschraubenfedern 22 (oder als Tellerfedern oder dergleichen) ausgebildet, die zwischen Filterhalteblech 19 und Muttern 24 auf die Bolzen 23 aufgezogen sind.

Wenn während des Sterilisiervorganges der sich schnell innerhalb der Sterilisierkammer aufbauende Druck den Behälterinnendruck um einen vorbestimmten Betrag überschreitet, wird durch diese Druckdifferenz das Filterblatt vom Deckel 12 ventilartig abgehoben und drückt dabei das Filterhalteblech 19 gegen die Kraft der zurückweichenden Federn 22. Bei Verringerung der Druckdifferenz führen die Federn 22 das Filterhalteblech und das Filterblatt 18 in die aus Fig.6 ersichtliche Dichtungsstellung zurück, in der der Strömungspfad wieder zwangsläufig durch das Filterblatt hindurch verläuft. Durch den sich kurzzeitig ergebenden Nebenschlußströmungspfad wird ein schneller Druckausgleich ermöglicht und eine Beschädigung von Filter oder Filterhalterung vermieden.

Der Sterilisierbehälter gemäß Fig. 5 entspricht in seinem Aufbau dem Sterilisierbehälter gemäß der DE-OS 33 40 963. Hier ist das Filterblatt 18 mittels eines Filterhaltebleches 19 gegen einen mit Perforationslöchern 16 versehenen Zwischendeckel 14 angedrückt, der am Rand einen U-förmigen Endflansch 26 aufweist, in dem ein Dichtring 28 eingelegt ist, der in Dichtungseingriff mit dem nach innen eingezogenen Oberrand 30 des Behälterunterteils 10 steht, wenn der Zwischendeckel über einen Innenspannverschluß 29 gegen den Behälterunterteil 10 vorgespannt ist. Dieser Zwischendeckel 14 ist von einem Behälterdeckel 12 umschlossen, der mit an seinem nach unten gezogenen Flanschrand befestigten Winkelstücken 13 auf der Oberseite des U-förmigen Endflansches 26 abgestützt ist. Diese Winkelstücke 13 sind über den Umfang verteilt und zwischen dem heruntergezogenen Rand des Deckels 12 und dem oberen Rand 30 des Behälterunterteils verläuft ein dem Strömungsaustausch dienender Ringspalt 40, der von den Winkelstücken 13 unterbrochen ist. Mit zwei Außenspannverschlüssen 42 ist der Behälterdeckel 12 unabhängig vom Zwischendeckel 14 am Behälterunterteil 10 festlegbar.

Die nachgiebige Festlegung des Filterhaltebleches 19 am Zwischendeckel 14 erfolgt über Federelemente 210, die über den Umfang verteilt angeordnet sind.

Diese Federelemente 210 bestehen aus Drahtfedern 32, die an ihrem einen Ende eine erste hohlnietartige Ausprägung 34 des Filterhaltebleches 19 umschließen und daran festgelegt sind, dann über eine entsprechende zweite Ausprägung 360 herumgeführt sind, und dann in einem schrägverlaufenden freien Federschenkel 380 nach den anderem Ende verlaufen, welches einen Verriegelungsabschnitt 39 und eine Handhabeöse 41 aufweist. Der Verriegelungsabschnitt greift hinter eine Abbiegung 44 jeweils eines Stützbleches 46, welches mit dem Rand des Zwischendeckels vernietet ist. Die Federelemente 210 sind demgemäß über den Verriegelungsabschnitt am Zwischendeckel abgestützt und drücken das Filterhalteblech mit einer vorbestimmten Kraft gegen den Zwischendeckel.

Wenn demgemäß der außerhalb des Sterilisierbehälters herrschende Druck einen vorbestimmten Wert überschreitet, hebt dieser das Filterblatt 18 und mit ihm das Filterhalteblech 19 vom Zwischendeckel ab, wobei sich die Federschenkel 380durchbiegen und es wird ein am Filterblatt 18 vorbeiführender Nebenstrompfad gebildet, der sofort wieder geschlossen wird, sobald der Druckausgleich erfolgt ist und der nach der Sterilisation zuverlässig geschlossen bleibt.

Die Federelemente 210können über die Handhabeöse 41 leicht betätigt werden, so daß das Filterhalteblech mit wenigen Handgriffen vom Zwischendeckel abgenommen werden kann , um das Filterblatt durch ein anderes zu ersetzen oder ein gebrauchtes zur Reinigung zu entnehmen.

## Patentansprüche

1. Sterilisierbehälter zur Aufnahme klinischen Sterilgutes, mit einem Unterteil (10), mit einem darauf dichtend aufsetzbaren Zwischendeckel (14, 14a), mit Filter (18) oder Ventilen (20) zur Ermöglichung des Medienaustausches Luft-Dampf während des Sterilisiervorganges -und mit einem den Zwischendeckel und den Oberrand des Behälterunterteils übergreifenden Behälterdeckel (12), der einen freien Strömungspfad in den Raum zwischen Behälterdeckel und Zwischendeckel definiert, und der mit dem Zwischendeckel lösbar verbunden ist, dadurch gekennzeichnet,
daß der Zwischendeckel (14, 14a) ohne Behälterdeckel (12) auf dem Behälterunterteil (10) durch Verschlüsse (29) steril dichtend verspannbar ist,
daß der Behälterdeckel (12) über dem aufgespannten Zwischendeckel am Behälterunterteil festlegbar ist,
und daß der Behälterdeckel (12) von dem auf dem Unterteil (10) aufgespannten Zwischendeckel (14, 14a) lösbar und abnehmbar ist.

2. Sterilisierbehälter nach Anspruch 1,
dadurch gekennzeichnet, daß der Zwischendeckel (14, 14a) auf dem Behälterunterteil (10) durch Verschlüsse (29) festlegbar ist, die zum Zwecke der Öffnung erst bei abgehobenem Behälterdeckel (12) zugänglich sind.

3. Sterilisierbehälter nach Anspruch 2, dadurch gekennzeichnet, daß der Behälterdeckelrand (38) den Verschluß (29) des Zwischendeckels übergreift.

4. Sterilisierbehälter nach einen der Ansprüche 1 bis 3, dadurch gekennzeichnet, daß zwischen dem perforierten Zwischendeckel (14) und einem perforierten Filterhalteblech (19) ein Dauerfilter oder ein Einwegfilter (18) mittels einer lösbaren Spannvorrichtung (21) eingespannt ist.

5. Sterilisierbehälter nach einem der Ansprüche 1 bis 4, dadurch gekennzeichnet, daß auf den perforierten Zwischendeckel (14) ein Dauerfilter oder ein Einwegfilter (18) auflegbar und mittels eines von den Verschlüssen (29) erfaßten Filterhalterahmens (48) oder eine Filterhalteblechs (19) festklemmbar ist.

6. Sterilisierbehälter nach einem der Ansprüche 1 bis 5, dadurch gekennzeichnet, daß an der Innenseite des Behälterdeckels (12) Niederhaltebleche (36) angeordnet sind, die bei aufgesetztem Behälterdeckel den Zwischendeckel (14) in Dichtungseingriff halten und das Absinken des Behälterdeckels (12) auf den Zwischendeckel (14, 14a) verhindern.

7. Sterilisierbehälter, insbesondere nach Anspruch 1 mit einem Filterblatt, das die Deckelöffnungen abdeckt und das sterilisierte Gut vor einer Reinfektion schützt, dadurch gekennzeichnet, daß das Filterblatt (18) beim Überschreiten eines vorbestimmten Druckdifferentials von den Deckelöffnungen (16) unter Freigabe eines Nebenstrompfades ventilartig abhebbar ist und nach Unterschreiten eines vorbestimmten Druckdifferentials die Deckelöffnung (16) erneut schließt.

8. Sterilisierbehälter nach Anspruch 7 mit einem mit Öffnungen versehenen Filterhalteblech, das das Filterblatt von innen her gegen den mit Öffnungen versehenen Deckel andrückt, dadurch gekennzeichnet, daß das Filterhalteblech (19) unter Federvorspannung steht und über Federelemente (210)am Deckel (12; 14) abgestützt ist, die als Drahtfedern (32) ausgebildet sein können.

9. Sterilisierbahälter nach Anspruch 8, dadurch gekennzeichnet, daß die Federelemente (210) das Filterhalteblech (19) lösbar mit dem Dekkel (12; 14) verbinden.

10. Sterilisierbehälter nach Anspruch 8, dadurch gekennzeichnet, daß die Drahtfedern mit einem mit ihrem einen Ende an Vorsprüngen (34; 360) des Filterhaltebleches (19) befestigt sind und mit ihrem anderen Ende (39) an Stützblechen (46) abgestützt sind, die vom Deckel (14) nach innen vorstehen.

11. Sterilisierbehälter nach den Ansprüchen 8 und 9, dadurch gekennzeichnet, daß die Federelemente von Druckschraubenfedern (22) oder Tellerfedern gebildet sind, die auf Deckel (12) und Filterhalteblechen (19) verbindende Befestigungstolzen (23) zwischen Filterhalteblech (19) und Spannmutter (24) aufgezogen sind.

12. Sterilisierbehälter nach Anspruch 9, dadurch gekennzeichnet, daß der mit dem ventilartig abhebbaren Filterblatt (18) versehene Deckel ein Behälterzwischendeckel (14) ist, der unter Bildung eines oder mehrerer Strömungspfade (40) von einem Behälterdeckel (12) umschlossen ist.

## Claims

1. A sterilizer container for storing sterilized clinical items comprising a base portion (10); an intermediate cover (14, 14a) being installed in sealing manner with said base portion, said intermediate cover being fitted with a filter (18) or with valves (20) allowing an exchange of air and steam therethrough whilst sterilisation is in progress; and a container cover (12) spaced to said intermediate cover and the upper edge of said base portion so that a free flow passage is defined in between the container cover and the intermediate cover in a detachable manner, characterized in that the intermediate cover (14, 14a) can be clamped in a sterile manner to the base portion (10) in a sealing manner therewith by a locking mechanism (29) without the container cover (12) connected thereto, that the container cover (12) can be connected to the base member above the intermediate cover clamped to the base portion, and that the container cover (12) is detachable from said intermediate cover (14, 14a) fixed to the base portion (10) and can be taken off from it.

2. A sterilizer container as claimed in claim 1, characterized by the intermediate cover (14,14a) being fixed to the base portion (10) by a locking mechanism ((29) accessible for opening purposes only after removal of the container cover (12).

3. A sterilizer container as claimed in claim 2, characterized by the edge (38) of the container cover overlapping said locking mechanism (29) for the intermediate cover.

4. A sterilizer container as claimed in one of the claims 1-3, charecterized by a permanent filter or a disposable filter (18) being spanned between the perforated intermediate cover (14) and a perforated filter supporting sheet (19) by means of a releasable clamping device (21).

5. A sterilizer container as claimed in claims 1 - 4, characterized in that a permanent filter or a disposable filter (18) can be placed on and can be clamped to the perforated intermediate cover (14) by means of a filter supporting frame (48) or a filter supporting sheet (19) engaged by said locking mechanism (29).

6. A sterilizer container as claimed in one of the claims 1-5, characterized in that strips (36) are provided on the inner side of the container cover (12) to hold down the intermediate cover (14) in sealing connection, and to prevent lowering of the container cover (12) onto the intermediate cover (14).

7. A sterilizer container especially as claimed in claim 1, comprising a filter sheet covering the perforations of the cover and protecting the sterilized items from recontamination, characterized in that the filter sheet (18) acts like a valve by opening a shunt flow path and by being detachable from the apertures (16) of the cover when a predetermined pressure differential is exceeded and that the apertures (16) are closed again as soon as the pressure is below a predetermined pressure differential.

8. A sterilizing container as claimed in claim 7 comprising a filter supporting sheet with openings pushing the filter sheet against the perforated cover from the inner side, characterized in that the filter supporting sheet is pressed against the cover (12;14) by spring elements (210) which may be wire springs.

9. A sterilizer container as claimed in claim 8, characterized in that said spring elements (210) are provided in a way that allows connecting or disconnecting of the filter supporting sheet (19) and the cover (12;14).

10. A sterilizer container as claimed in claim 8, characterized in that one of the ends of the wire springs are connected to projections (34;360) of the filter supporting sheet (19) and are supported with their other ends by supporting sheets (46) projecting inwards from the cover (14).

11. A sterilizer container as claimed in claims 8 and 9, characterized in that the spring elements comprise helical pressure springs (22) or cup springs which are surrounding the tighten bolts (23) between the filter supporting sheet (19) and a clamping nut (24), whereby said bolts connect the cover (12) with the filter supporting sheet (19).

12. A sterilizer container as claimed in claim 9, characterized in that the cover comprising the valve like filter sheet (18) constitutes an intermediate cover (14) enclosed by the container cover (12) with one or several flow paths between them.

## Revendications

1. Récipient de stérilisation destiné à recevoir une substance stérile clinique, comprenant une partie inférieure (10) ; un couvercle intermédiaire (14, 14a) pouvant être placé hermétiquement sur cette dernière, et muni d'un filtre (18) ou de valves (20) pour permettre l'échange air- vapeur au cours du processus de stérilisation ; et un capot (12) qui coiffe le couvercle intermédiaire et le bord supérieur de la partie inférieure du récipient, définit un trajet d'écoulement libre dans l'espace situé entre le capot et le couvercle intermédiaire, et est relié amovi- blement audit couvercle intermédiaire, caractérise par le fait que le couvercle intermédiaire (14, 14a) peut être bloqué de manière étanche, sans capot (12), sur la partie inférieure (10) du récipientode manière stérile au moyen d'obturations (29) ; par le fait que le capot (12) peut être assujetti à la partie inférieure du récipient, au-dessus du couvercle intermédiaire bloqué ; et par le fait que le capot (12) peut être séparé et enlevé du couvercle intermédiaire (14, 14a) bloqué sur la partie inférieure. (10).

2. Récipient de stérilisation selon la revendication 1,
caractérisé par le fait que le couvercle intermédiaire (14, 14a) peut être verrouillé, sur la partie inférieure (10) du récipient, au moyen d'obturations (29) accessibles, en vue de l'ouverture, uniquement lorsque le capot (12) est soulevé.

3. Récipient de stérilisation selon la revendication 2, caractérisé par le fait que le rebord (38) du capot emprisonne par-dessus l'obturation (29) du couvercle intermédiaire.

4. Récipient de stérilisation selon l'une des revendications 1 à 3, caractérisé par le fait qu'un filtre permanent ou un filtre jetable (18) est enchâssé, au moyen d'un dispositif amovible de blocage (21), entre le couvercle intermédiaire perforé (14) et une tôle perforée (19) de retenue du filtre.

5. Récipient de stérilisation selon l'une des revendications 1 à 4, caractérisé par le fait qu'un filtre permanent ou un filtre jetable (18) peut être placé sur le couvercle intermédiaire perforé (14) et peut être coincé fermement au moyen d'un châssis (48) de retenue du filtre, capturé par les obturations (29), ou au moyen d'une tôle (19) de retenue du filtre.

6. Récipient de stérilisation selon l'une des revendications 1 à 5, caractérisé par le fait que des tôles (36) de compression vers le bas, disposées à la face interne du capot (12), maintiennent le couvercle intermédiaire (14) en prise étanche lorsque ledit capot est mis en place, et empêchent l'affaissement dudit capot (12) sur ledit couvercle intermédiaire (14, 14a).

7. Récipient de stérilisation notamment selon la revendication 1, doté d'une feuille filtrante qui recouvre les orifices du couvercle et protège la substance stérilisée d'une réinfection, caractérisé par le fait que, lors du dépassement positif d'une différence de pression prédéterminée, la feuille filtrante (18) peut être soulevée à l'écart des orifices (16) du couvercle à la manière d'une soupape, en dégageant un trajet d'écoulement secondaire, et obture de nouveau les orifices (16) du couvercle après dépassement négatif d'une différence de pression prédéterminée.

8. Récipient de stérilisation selon la revendication 7, équipé d'une tôle de retenue du filtre qui est percée d'orifices et presse la feuille filtrante, de l'intérieur, contre le couvercle percé d'orifices,
caractérisé par le fait que la tôle (19) de retenue du filtre est soumise à une précharge élastique et prend appui, contre le couvercle (12 ; 14), par l'intermédiaire d'éléments élastiques (210) pouvant être réalisés sous la forme de ressorts (32) en fil métallique.

9. Récipient de stérilisation selon la revendication 8,
caractérisé par le fait que les éléments élastiques (210) assurent la liaison libérable de la tôle (19) de retenue du filtre avec le couvercle (12 ; 14).

10. Récipient de stérilisation selon la revendication 8,
caractérisé par le fait que les ressorts en fil métallique sont fixés, par l'une de leurs extrémités, à des protubérances (34 ; 360) de la tôle (19) de retenue du filtre et prennent appui, par leur autre extrémité (39), contre des tôles de soutien (46) faisant saillie vers l'intérieur à partir du couvercle (14).

11. Récipient de stérilisation selon les revendications 8 et 9, caractérisé par le fait que les éléments élastiques sont formés par des ressorts hélicoïdaux de pression (22) ou par des rondelles élastiques enfilé(e)s, entre la tôle (19) de retenue du filtre et un écrou de blocage (24), sur des chevilles de fixation (23) solidarisant le capot (12) et les tôles (19) de retenue du filtre.

12. Récipient de stérilisation selon la revendication 9,
caractérisé par le fait que le couvercle, pourvu d'une feuille filtrante (18) soulevable à la manière d'une soupape, est un couvercle intermédiaire (14) du récipient qui est entouré, par un capot (12), en formant un ou plusieurs trajet(s) d'écoulement (40).
